(19) Europäisches Patentamt
European Patent Office
Office européen des brevets

(11) Publication number: 0 195 749
A2

(12) **EUROPEAN PATENT APPLICATION**

(21) Application number: 86830012.0

(22) Date of filing: 17.01.86

(51) Int. Cl.[4]: A 61 K 31/415

(30) Priority: 22.01.85 IT 1917985

(43) Date of publication of application: 24.09.86 Bulletin 86/39

(84) Designated Contracting States: AT BE CH DE FR GB LI LU NL SE

(71) Applicant: AZIENDE CHIMICHE RIUNITE ANGELINI FRANCESCO A.C.R.A.F. S.p.a.
Viale Amelia, 70
I-00181 Roma(IT)

(72) Inventor: Silvestrini, Bruno
Via M. Schipa n. 15
I-00179 Rome(IT)

(74) Representative: La Ciura, Salvatore
Via Francesco Sforza 5
I-20122 Milano(IT)

(54) **Use of benzydamine in the treatment of the trichomonas vaginalis and gardnerella vaginalis infections.**

(57) The present invention refers to a new utilization of benzydamine, consisting in the treatment of the Trichomonas vaginalis and Gardnerella vaginalis infections.

EP 0 195 749 A2

Croydon Printing Company Ltd.

"USE OF BENZYDAMINE IN THE TREATMENT OF THE TRICHOMONAS VAGINALIS AND GARDNERELLA VAGINALIS INFECTIONS"

## INTRODUCTION

Benzydamine is a drug having the following structural formula:

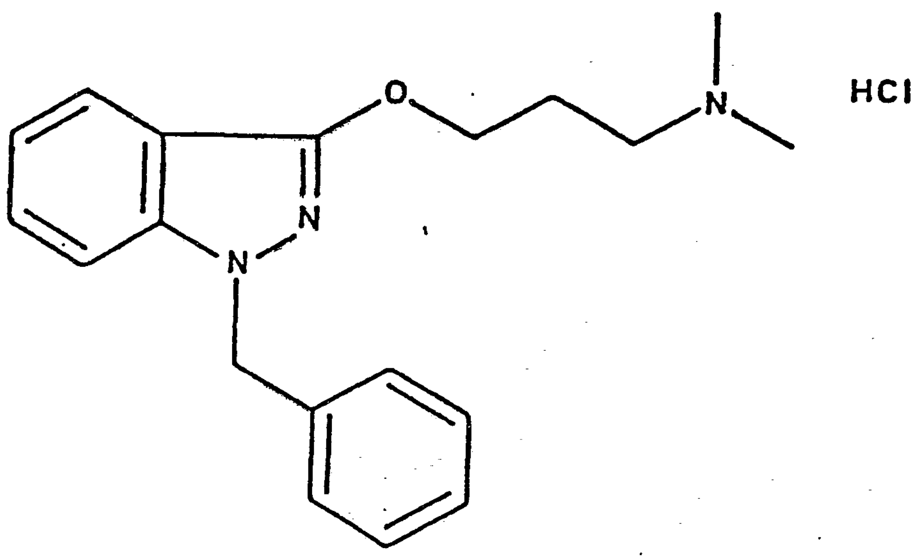

By a previous Italian Patent Application (Ser. No. 16898/63, filed on August 9, 1963), then extended to other countries, inclusive of the U.S. (US Patent No. 3 381 905), there have been claimed the analgesic, antiphlogistic and muscle relaxant properties of such product. The analgesic and antiphlogistic properties of benzydamine have been subsequently confirmed and studied in detail, both in the laboratory animal and in humans (MEGA M. et al. Clin. Exp. Obstet. Gynecol. 4,19-27, 1977; FROOM J. et al. Curr. Ther. Res. 26,856-861, 1979; KOPERA M. Inflammation Proc. Int. Symp., Bologna 1967 - Eds. SILVESTRINI et al. Excerpta Med. Foundation, Amsterdam, 100-106,1968; DELL'ORTO R. et al. Clin. Europ. 7,296-303, 1968; LISCIANI R. et al. Europ. J. Pharmacol. 3,157-162, 1968; SILVESTRINI B. et al. Arzneimittel-Forsch. 16,59-63, 1966).

Benzydamine is actually therapeutically employed in many countries, both by oral and by topic administration, as an anti-inflammatory medicine.

The above mentioned studies led to the discovery of the specific activity of benzydamine against Trichomonas vaginalis and Gardnerella vaginalis.

PRIOR ART

Although benzydamine is presently employed also in the treatment of inflammatory vaginites, no one had ever imagined that its therapeutical effects could derive from a specific action either on Trichomonas vaginalis or on Gardnerella vaginalis. In this respect special attention should be paid to a previous clinical research on the trichomoniasis vaginalis, wherein a specific therapy was studied both alone and in association with benzydamine (MARCOLIN D. et al. Clin. Exp. Obstet. Gynecol. 6,50-53, 1979). The associated therapy proved better than the specific therapy alone, but this result

was put down to the analgesic-antiphlogistic properties of benzydamine. Accordingly, not even the present study did surmise that benzydamine could have the microbiological properties claimed by this patent.

The prior art concerning benzydamine includes also some research relevant to the antibacterial and antifungal action it exerts at concentrations comprised between 0.1 and 0.015% (SILVESTRINI B. et al. Arch. Int. Pharmacodyn. 163,61, 1966; SILVESTRINI B. et al. Boll. Chim. Farm. 107, 353-361, 1968; TAGLIAPIETRA L., Boll. Chim. Farm. 109, 374-379, 1970). Such an activity may be defined of the disinfectant kind, as it is exerted aspecifically both toward bacteria and toward fungi. Besides, it requires relatively high concentrations.

Because of these two reasons, it should be regarded as fully different from that directed towards the Trichomonas vaginalis and Gardnerella vaginalis, claimed by the present patent.

## DESCRIPTION OF THE INVENTION

The Trichomonas vaginalis is a protozoan causing infections of the genitourinary tract both in man and in woman. It has an oval shape, a lenght ranging from 5 to 30$\mu$, a likewise oval nucleus and four distal hamate flagella. Its capability to multiply is so developed that within few days millions of protozoans may be present in the vagina or in the male genital apparatus.

The Trichomonas vaginalis is present in a high percentage of vaginal secretions, that according to our experience reaches 25%, even though it does not always

cause apparent troubles. These show up presumably under the action of concurrent factors, capable of making said protozoan virulent, or of breaking down the natural defenses. The propagation of the infection is mainly connected with the sexual contagion. The symptoms consist in an increase in the secretions, that become often liquid, ill-smelling and - not seldom - foamy, as well as in pruritus and burning sensation, sometimes affecting also the anal region. The mucosa looks reddened, sometimes granulous, with small sores. What often complicates the clinical picture is the fact that the Trichomonas vaginalis alters the physiological bacterial flora, favouring thus the setting in of overinfections both of bacterial and of fungous type.

The Trichomonas vaginalis infections are strongly rebellious to the therapy, and show a tendency to reappear in a more serious form. For this reason the search for new cures is of great medical interest.

The antiprotozoonal activity peculiar to benzydamine was studied in vitro on freshly insulated Trichomonas vaginalis.

To this end was employed material taken from 12 female patients who, upon direct examination of the vaginal secretion effected by phase-contrast microscope, turned out to be infected.

The protozoans were kept at 37°C in Diamond medium, with 10% addition of inactivated horse serum, at pH 6.1 (DIAMOND L. J. Parasitol., 43, 488-490, 1957). 48 h cultures of Trichomonas vaginalis were diluted over a medium containing benzydamine at scalar concentrations.

The antiprotozoonal effect was checked microscopically 48 hours later.

The Gardnerella vaginalis is a gram-variable, small, pleomorph bacillus, forming colonies of 0.25-0.44 mm diameter. It has a beta-hemolytic action on human or rabbit blood; the microorganism is catalase- and oxidase- negative, hippurate-positive, and may hydrolize starch and maltose with production of acid (Piot B. et al., J.Clin.Microbiol., 1982, 15, 19-24). In order to distinguish it from other vaginal bacteria, the metronidazole- and sulfonamide- sensitivity test may be employed (Bailey R.K. et al., J.Clin.Microbiol., 1979, 9: 65-71). To study the Gardnerella vaginalis was employed the selective medium for insulation propounded by Ison et al. (Ison, C.A. et al. J.Clin. Path., 1982, 35, 550-554), supplied by Oxoid.

The medium was enriched with rabbit blood. In order to insulate the Gardnerella vaginalis, the medium was inoculated on the surface with vaginal secretion taken from women whose symptoms were as follows:

1. Ill-smelling secretion;
2. Subjective troubles, such as pruritus and reddening;
3. Lack, on microbiological examination, of other common pathogenic germs;
4. Presence of clue cells in the fresh specimens, that is epithelial cells with granulations or dotting caused by gram-negative or gram-positive bacteria adherent to the cell surface.

The identity of the Gardnerella vaginalis was further checked both through the fermentation tests and by the

employ of disks containing metronidazole and sulfonamide, at the doses of 50 and 1000 µg respectively.

With a view to establishing the specific effects on Trichomonas vaginalis and on Gardnerella vaginalis the antibacterial and antifungous activity of benzydamine on some representative microorganisms was also studied, making use of the experimental conditions previously described (SILVESTRINI B. et al. 1966).

The results obtained are set out in Table 1.

Benzydamine displays an activity against Trichomonas vaginalis and Gardnerella vaginalis at concentrations in the region of 10-20 µg/ml, while its activity against fungi and the other bacteria manifests iteself only at concentrations that are 10-20 times as great.

The clinical research was carried out as follows. The patients of female sex who, at the medical examination, complained of vaginal troubles, accompanied with an abnormal secretion and pruritus, were sistematically subjected to the microscopical examination of the vaginal secretion. Thus, it was possible to ascertain the presence of Trichomonas vaginalis and Gardnerella vaginalis in the secretion of 40 and 20 patients, respectively.

The first 40 patients were divided out in homogeneous groups made up each of 10 patients, who were treated as follows:

A. Three applications a day, in the form of intravaginal washing made with a standard irrigator, of a 15% benzydamine aqueous solution in isotonic solution of sodium chloride.

B. Three applications a day, according to the above mentioned method, of an isotonic aqueous solution of sodium chloride.

C. Three applications a day of 2-3 g of a 0.5% Benzydamine ointment, containing the following eccipients per 100 g:

- 18 g glycerol stearate
- 5 g decyl oleate
- 3 g polyoxyethylenated oleic glycerides
- 2 g cetylstearyl alcohol

TABLE 1

ACTIVITY OF BENZYDAMINE ON SOME MICROORGANISMS

| Microorganisms | pH | Studied concentrations (µg/ml) and activities (+ – M.C.I.) | | | | | | | | | | |
|---|---|---|---|---|---|---|---|---|---|---|---|---|
| | | 10 | 20 | 40 | 50 | 70 | 80 | 100 | 150 | 200 | 400 | 500 |
| Trichomonas vaginalis | 6,1 | + | + | + | + | + | + | + | + | + | + | + |
| Gardnerella vaginalis | 7 | ± | ± | + | + | + | + | + | + | + | + | + |
| Aspergillus niger | 6,5 | – | – | – | – | – | – | – | – | + | + | + |
| Candida albicans | 6,5 | – | – | – | – | – | – | – | – | + | + | + |
| Escherichia choli | 6,5 | – | – | – | – | – | – | – | + | + | + | + |
| Staphilococcus aureus | 6,5 | – | – | – | – | – | – | – | – | ± | + | + |

- 1 g fluid silicone
- 5 g propylen glycol
- 0.18 g methyl p-hydroxybenzoate
- 0.02 g propyl p-hydroxybenzolate
- purified water q.s. to 100 g

D. Three applications a day, according to the above described method, of an ointment containing the eccipients only.

The study was performed under double-blind conditions. The treatments were carried out for two weeks. The microbiological exam of the vaginal secretion and the clinical remarks were carried out at the very beginning, as well as 7 and 14 days after the beginning of the treatments; they were further repeated also 15 days later in order to establish relapses - if any - in the patients who had reacted to the treatments. The results of the microbiological exams are set out in Table 2 hereunder.

TABLE 2

Effects of benzydamine on the presence of Trichomonas vaginalis in the vaginal secretion.

| Treatments | N° Patients | Presence of Trichomonas vaginalis in the vaginal secretion<br>Time in weeks<br>0 | 1 | 2 | 4 |
|---|---|---|---|---|---|
| Benzydamine 0.5% as ointment | 10 | 10/10 | 0/10 | 0/10 | 0/10 |
| Reference | 10 | 10/10 | 9/10 | 8/10 | 9/10 |
| Benzydamine 0.15% as washing | 10 | 10/10 | 5/10 | 1/10 | 1/10 |
| References | 10 | 10/10 | 10/10 | 10/10 | 9/10 |

In the group treated with the 0.5% benzydamine ointment the search for Trichomonas vaginalis proved negative already after the first week of treatment, and maintained as such also on subsequent examinations, while the reference group revealed 1, 2 and 1 negative patients after 1, 2 and 4 weeks respectively. The fact that a patient who was negative after the second week of treatment resulted to be positive again after 4 weeks was presumably to ascribe to a technical mistake. An antiprotozoonal effect was likewise observed in the patients treated with the 0.15% benzydamine solution, even though to a less noticeable extent than with the ointment treatment.

The results obtained through the patients' clinical examination are shown in Table 3 hereunder.

## TABLE 3

Effects of benzydamine on the clinical picture of the Trichomonas vaginalis infection.

| Treatments | N° Patients | Detected symptoms (1) | Time in weeks 0 | 1 | 2 | 4 |
|---|---|---|---|---|---|---|
| Benzydamine 0.5% as ointment | 10 | Inflammation | + + | + | ± | ± |
| | | Secretion | + + + | + | + | 0 |
| | | Pain | + + | + | ± | 0 |
| References | 10 | Inflammation | + + | + + | + + | + + |
| | | Secretion | + + + | + + + | + + + | + + |
| | | Pain | + + | + + | + + | + |
| Benzydamine 0.15% as washing | 10 | Inflammation | + + | ± | 0 | 0 |
| | | Secretion | + + + | + | ± | 0 |
| | | Pain | + + | + | 0 | 0 |
| References | 10 | Inflammation | + + | + + | + + + | + + |
| | | Secretion | + + + | + + | + + + | + + |
| | | Pain | + + | + + | + + | + + |

(1) The symptoms have been assessed according to the following criteria: 0, absent; +, plain; ++, very sharp; the average value is quoted.

The best results were achieved by the 0.15% benzydamine aqueous solution, that gave decidedly better results in respect to the reference group.

The ointment containing 0.5% benzydamine revealed too a clear therapeutic action, in comparison to the reference group.

These tests were carried out on female patients in whom, due also to anatomic reasons, the Trichomonas vaginalis infection is especially difficult to eradicate. Similar or even better therapeutical results can also be obtained on male patients.

As concerns the active concentrations, the results obtained in vitro show that it is possible to achieve successful results with concentrations comprised between 0.1 and 1%.

The clinical study of the effect of benzydamine on the Gardnerella vaginalis was carried out on 20 patients, who were found to be infected with Gardnerella vaginalis twice at three days' distance. Ten patients were treated with the above mentioned benzydamine-containing ointment, and other ten with the ointment containing the eccipients only.

The test was carried out under the conditions employed for the study on Trichomonas, except for the duration of the treatment, which was of 7 days only. The results of the microbiological examinations are shown in Table 4.

TABLE 4

Effects of benzydamine on the presence of Gardnerella vaginalis in the vaginal secretion.

| Treatments | N° Patients | Presence of Gardnerella vaginalis in the vaginal secretion<br>Time in weeks | | |
|---|---|---|---|---|
| | | 0 | 1 | 2 |
| Benzydamine 0.5% | 10 | 10 | 0 | 0 |
| Reference | 10 | 10 | 10 | 7 |

The effect of the treatment was even sharper than in the case of the Trichomonas vaginalis infection: in fact within a week the tests revealed that the Gardnerella was not present in any cases. Likewise, all the patients treated with benzydamine showed a clear attenuation of the clinical symptoms of the infection.

0195749

## CLAIMS

1. A method for the treatment of the Trichomonas vaginalis infections, both in the male and in the female, based on topical applications of benzydamine.

2. A method for the treatment of the Gardnerella vaginalis infections, based on topical applications of benzydamine.

3. A method according to the claims 1 and 2, wherein benzydamine is used at concentrations in the region of 0.1-1%.

4. A method according to the claims 1, 2 and 3, wherein there are employed farmaceutical formulations suitable for the topical use and consisting in aqueous solutions, ointments and the like.